# EUROPÄISCHE PATENTSCHRIFT

(11) **EP 4 321 110 B1**
(45) Veröffentlichungstag und Bekanntmachung des Hinweises auf die Patenterteilung: **18.11.2026**
(21) Anmeldenummer: 23190854.2
(22) Anmeldetag: 10.08.2023
(51) Int. Cl.: A61B 17/16

(54) **SCHAFTKUPPLUNG UND ZUGEHÖRIGES MONTAGEVERFAHREN**
SHAFT COUPLING AND CORRESPONDING ASSEMBLY METHOD
ACCOUPLEMENT DE TIGE ET PROCÉDÉ D'ASSEMBLAGE ASSOCIÉ

(30) Priorität: 11.08.2022 DE 102022120351
(43) Veröffentlichungstag der Anmeldung: 14.02.2024
(73) Patentinhaber: Aesculap AG, 78532 Tuttlingen (DE)
(72) Erfinder: BUERK, Andre, 78056 Villingen-Schwenningen (DE); VOGLER, Aaron, 88637 Leibertingen (DE)
(74) Vertreter: Winter, Brandl - Partnerschaft mbB

(56) Entgegenhaltungen:
- DE-A1- 102015 110 415

## Beschreibung

### Technisches Gebiet

Die vorliegende Offenbarung betrifft eine Schaftkupplung, insbesondere eine Schaftkupplung für ein oder eines medizinischen Motorhandstücks mit einem abnehmbaren Schaft, der einen motorangetriebenen Endeffektor trägt.

In der modernen minimalinvasiven Chirurgie werden Motorhandstücke für die Bedienung von Werkzeugen/Instrumenten beispielsweise zur Bearbeitung von Knochen, Knorpeln bei arthroskopischen Eingriffen, in der Wirbelsäulenchirurgie und dergleichen orthopädischen/chirurgischen Behandlungen sowie zur Bearbeitung von organischem Material in der Neurochirurgie verwendet. Die Endeffektoren, wie beispielsweise Fräser, Drehmesser, Polierköpfe oder Ähnliches sind in der Regel austauschbar. Der Endeffektor ist dabei vorzugsweise in einem Schaft des Werkzeugs/Instruments an dessen distalem Ende gelagert. Beispiele für derartige Motorhandstücke mit auswechselbaren medizinischen Werkzeugen sind in den Dokumenten 10 2012 108 264 A1, DE 103 11 455 B3 und US 5,893,851 B gezeigt. Kupplungen für koaxiale Antriebswellenabschnitte sind in den Dokumenten US 6,062,575 B und US 2017/0071608 A1 gezeigt, wobei im letzteren Dokument ein das Werkzeug aufnehmender Schaft über eine Bajonettverbindung am Handstück fixierbar ist.

Aus dem Dokument DE 10 2015 110 415 A1 ist ein Chirurgie-Handgriff zum drehfesten Aufnehmen eines Handstückschafts bekannt, wobei eine Schaftkupplung gemäß dem Oberbegriff des Patentanspruchs 1 vorgesehen ist. Auch aus dem Dokument US 5,522,669 ist allgemein eine derartige Schaftkupplung bekannt.

Als Werkzeugantrieb ist je nach Verwendungszweck und beabsichtigter Werkzeugdrehzahl ein hydraulischer, pneumatischer, oder elektromotorischer Antrieb vorgesehen, der über einen Drehmoment-Übertragungszug innerhalb des Handinstruments bzw. des Griffabschnitts mit dem Werkzeugkopf (Endeffektor) wirkverbunden ist. Das Motorhandstück hat aus Bedienungsgründen eine im Wesentlichen kreiszylindrische Form und es ist oftmals zweiteilig mit einem den Antrieb aufnehmenden Motorgehäuseteil und einem daran koaxial angesetzten Griffteil aufgebaut. Am Griffteil befinden sich häufig auch Bedienungselemente, mit denen verschiedene Zusatzfunktionen des Instruments, wie z.B. eine Abwinklung einer den Endeffektor tragenden Schaftspitze oder das Auswerfen des Endeffektors, gesteuert werden können.

Dies bedeutet, dass der Aufbau eines solchen Motorhandstücks recht komplex wird, weil die für den Antrieb des Endeffektors und für die Steuerung der Zusatzfunktionen benötigten Komponenten und Mechanismen auf radial sehr begrenztem Raum untergebracht werden müssen. Weil ferner der Außendurchmesser des Handstücks zur Bereitstellung eines guten Handhabungsgefühls begrenzt ist, und weil das Zentrum des Handstücks vom Antriebsstrang und gegebenenfalls für einen Auswurfmechanismus für den Endeffektor benötigt wird, verbleibt nur ein recht kleiner radialer Bauraum, um die Mimik für die Ansteuerung der Zusatzfunktionen unterzubringen.

Verschärft wird diese Problematik dadurch, dass es oftmals erwünscht ist, den Schaft mit einfachsten Handgriffen vom Motorhandstück abnehmen zu können, wobei auch hier ein möglichst einfacher Betätigungsmechanismus vorgesehen sein soll, der durch einen Bediener intuitiv und sicher bedient werden kann.

### Gegenstand der Offenbarung

Der Offenbarung liegt deshalb die Aufgabe zugrunde, eine Schaftkupplung zu schaffen, die es ermöglicht, bei geringstem benötigten radialen Bauraum zwei konzentrisch ineinander gefügte kreiszylindrische Komponenten lösbar miteinander zu koppeln und mit einfachsten Handgriffen zu entkoppeln, so dass sie sich in besonderer Weise für die Ankopplung eines Schafts für einen motorangetriebenen Endeffektor an ein Motorhandstück eignet. Eine weitere Aufgabe besteht darin, ein einfaches Montageverfahren für eine derartige Schaftkupplung bereit zu stellen.

Diese Aufgabe wird hinsichtlich der Schaftkupplung durch die Merkmale des Patentanspruchs 1 und hinsichtlich des Montageverfahrens durch die Merkmale des Patentanspruchs 10 gelöst.

Die Besonderheit der Schaftkupplung ist darin zu sehen, dass alle benötigten Komponenten in einem recht schmalen radialen Bauraum radial außerhalb der kreiszylindrischen Aufnahme des Gehäuses, wie z.B. eines Motorhandstücks eines medizinischen Instruments, Platz finden. Weil der Schließkörper als Schließhülse ausgebildet ist, die zusammen mit der Druckfeder auf der Außenseite der kreiszylindrischen Aufnahme geführt sitzt, kann ihm bei geringer Wandstärke eine ausreichend gute Stabilität gegeben werden. Mit der Mitnehmerzunge auf der vom Schaft weg gerichteten Seite gelingt es, einen oftmals, beispielsweise bei einem Motorhandstück eines medizinischen Instruments bedienungstechnisch vorgegebenen, erheblichen axialen Abstand zu einem Betätigungselement der Schaftkupplung zu überbrücken. Gleichzeitig wird der zumindest einen Mitnehmerzunge die Funktion übertragen, die Schließhülse in einer bestimmten Drehstellung zu fixieren und axial zu führen. Somit kann es der Schließhülse bei einfacher Montage ermöglicht werden, in einer ersten Drehstellung auf die Außenseite der kreiszylindrischen Aufnahme aufgefädelt und in einer zweiten Drehstellung drehfest fixiert zu werden. Dadurch, dass die Schließhülse eine der Anzahl der Verriegelungskörper entsprechende Anzahl von Montage-Ausnehmungen aufweist, die nur in der ersten Drehstellung der Schließhülse in Umfangsrichtung mit den Führungsöffnungen fluchten, kann das Einlegen des zumindest einen Verriegelungskörpers erfolgen, ohne die Druckfeder der Schließhülse belasten zu müssen. Denn die Montage-Ausnehmungen sind nur wirksam in der ersten Drehstellung der Schließhülse und sie verlieren ihre Funktion in der zweiten Drehstellung. Auf diese Weise wird die Montage der Schaftkupplung besonders einfach, mit der Besonderheit, dass die Kupplungskomponenten bei der Montage weitestgehend geschont bleiben.

Die Montageverfahren sind Gegenstand der Ansprüche 10 bis 13.

Vorteilhafte Ausgestaltungen und Anordnungen der Montage-Ausnehmungen sind Gegenstand der Ansprüche 8 und 9.

Eine besonders einfache Herstellung der Schließhülse ergibt sich dann, wenn die zumindest eine Montage-Ausnehmung in der Schließhülse von einer in einem Schulterabschnitt innenseitig ausgebildeten und axial verlaufenden Nut gebildet ist, die ausreichend tief ist, um den Schulterabschnitt der Schließhülse axial über den zugeordneten, bereits in der zylindrischen Aufnahme montierten Verriegelungskörper zu schieben. Bei der Montage ist lediglich darauf zu achten, dass die axial verlaufende Nut in Fluchtung mit dem von der Außenoberfläche der kreiszylindrischen Aufnahme um ein kleines Maß vorstehenden Verriegelungskörper gebracht wird. Zur Positionierung der Schließhülse in dieser Drehstellung kann dabei in vorteilhafter Weise die Mitnehmerzunge herangezogen werden.

Wenn der zumindest eine Verriegelungskörper erst bei bereits aufgefädelter Schließhülse montiert werden soll, wird die zumindest eine Montageausnehmung von einer jeweils einem Verriegelungskörper zugeordneten Befüllöffnung gebildet, durch die in der ersten Drehstellung der Schließhülse jeweils ein Verriegelungskörper (90) radial in die Führungsöffnung der zylindrischen Aufnahme einsetzbar ist. Auch hier kann die zumindest eine Mitnehmerzunge zur Ausrichtung der Schließhülse auch in der ersten Drehstellung herangezogen werden.

Vorteilhafte Weiterbildungen sind Gegenstand der Unteransprüche.

Wenn die Schließhülse in der zweiten Drehstellung drehfest im Gehäuseabschnitt eines Gehäuses fixierbar ist, kann der Drehwinkel zwischen der ersten und der zweiten Drehstellung auf ein Minimum begrenzt werden.

Eine besonders effektive Drehlagensicherung der Schließhülse ergibt sich dann, wenn sich die zumindest eine Mitnehmerzunge durch einen zugehörigen Durchbruch in einem Ringflansch der kreiszylindrischen Aufnahme erstreckt und in der zweiten Drehstellung mittels eines radial von außen einsetzbaren Passteils in einer Anlagestellung an einer Seitenwand des Durchbruchs fixierbar ist. Aufgrund der Einsetzbarkeit von radial außen wird die Montage besonders einfach. Außerdem ergibt sich durch das Einsetzen der Passteile radial von außen der besondere Vorteil, dass die Passteile mit der Seitenwand des Durchbruchs formschlüssig verzahnt werden können. Auf diese Weise können die Passteile allein durch ihre Form dafür sorgen, dass sie nach dem Einlegen für die Schließhülse eine definierte seitliche Führung bereitstellen.

Vorteilhafterweise bildet die Schließhülse innenseitig zwei axial hintereinanderliegende Funktionsabschnitte mit unterschiedlichen Innendurchmessern aus. Auf diese Weise kann die Schließhülse durch eine einfache Herstellung der Innenkontur für ein gleichzeitiges Freigeben mehrerer über den Umfang verteilter Verriegelungskörper sorgen.

Wenn sich die zumindest eine Mitnehmerzunge von einem Schulterabschnitt eines die Funktionsabschnitte ausbildenden Schließhülsenkörpers weg erstreckt, und der Schulterabschnitt radial innerkalb der Mitnehmerzunge eine Anlagefläche für die Druckfeder ausbildet, ergibt sich ein besonders günstiger Krafteinleitungspunkt, so dass die Schließhülse wirksam vor einem Verkanten gesichert ist.

Grundsätzlich genügt für die Schaftkupplung ein einziger Verriegelungskörper und eine einzige Mitnehmerzunge. Die Schaftkupplung arbeitet aber mit besserer Funktionalität, wenn mehrere über den Umfang vorzugsweise gleichmäßig verteilte Verriegelungskörper und/oder Mitnehmerzungen vorgesehen sind.

Eine besondere Leichtgängigkeit der Schaftkupplung wird dann erzielt, wenn der zumindest eine Verriegelungskörper als Kugel ausgebildet ist.

### Beschreibung der Ausführungsbeispiele

Die Offenbarung wird nachfolgend anhand bevorzugter Ausführungsbeispiele mit Hilfe von Figuren näher erläutert. Es zeigen:
- Fig. 1: eine perspektivische Ansicht des distalen Abschnitts eines medizinischen Handinstruments;
- Fig. 2: eine weitere perspektivische Ansicht des distalen Abschnitts des medizinischen Handinstruments;
- Fig. 3: eine perspektivische Ansicht des Handstücks für das medizinische Handinstrument mit verriegelter Schaftkupplung;
- Fig. 4: eine perspektivische Ansicht des Handstücks für das medizinische Handinstrument mit entriegelter Schaftkupplung;
- Fig. 5: eine perspektivische Ansicht des distalen Abschnitts des Handinstruments mit entnommenem Schaft;
- Fig. 6: eine Teillängsschnittansicht der sich gegenüberliegenden Endabschnitte von Schaft und Handstück vor dem Kupplungsvorgang;
- Fig. 7: eine Teillängsschnittansicht des Handstücks bei eingekuppeltem Schaft;
- Fig. 8: in vergrößertem Maßstab eine Perspektivansicht des Motorhandstücks des medizinischen Handinstruments in einer anfänglichen Montagesituation einer Schließhülse;
- Fig. 9: eine Stirnansicht der in Figur 8 gezeigten Schließhülse vom distalen Ende aus betrachtet;
- Fig. 10 bis 12: Ansichten der die Schaftkupplung darstellenden Komponenten in aufeinanderfolgenden Montageschritten;

- Fig. 13: eine der Figur 7 entsprechende Schnittansicht des Handstücks bei geringfügig verdrehter Schnittebene bei abgenommenem Schaft; und
- Fig. 14: in vergrößertem Maßstab die Stirnansicht einer Variante der Schließhülse.

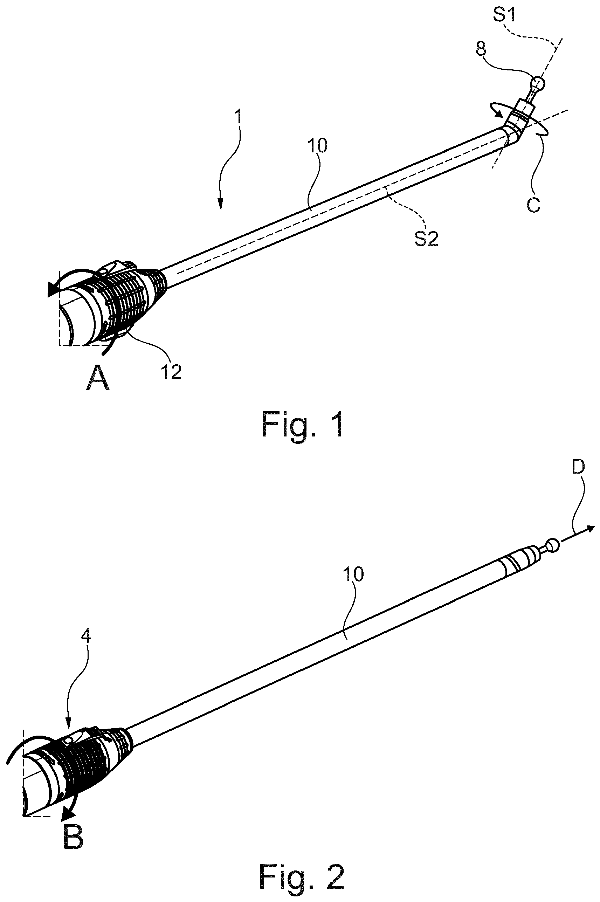

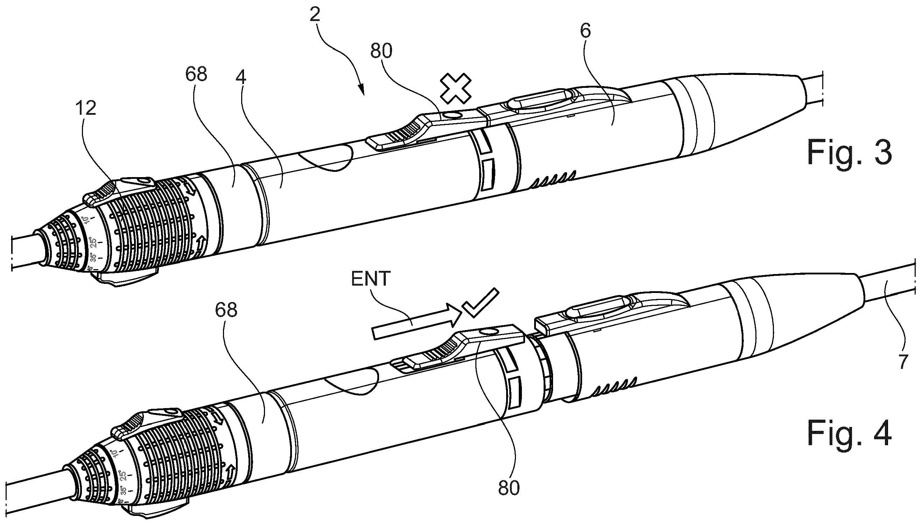

Figur 1 zeigt perspektivisch ein medizinisches Handinstrument 1 gemäß einer Ausführungsform der vorliegenden Offenbarung, bei der die erfindungsgemäße Schaftkupplung in vorteilhafter Weise einsetzbar ist. Das medizinische Handinstrument 1 weist dabei ein in den Figuren 3 und 4 gezeigtes offenbarungsgemäßes medizinisches Motorhandstück 2 auf, welches einen distalen Handgriffabschnitt 4 aufweist, der koaxial an eine Antriebseinheit 6 angeschlossen ist. Ein Kabel für die Stromversorgung der Antriebseinheit ist mit 7 bezeichnet.

Ferner weist das Handinstrument 1 ein distales Werkzeug (Endeffektor/Effektorabschnitt) 8 auf, welches über einen (Werkzeug-)Schaft 10 mit dem Motorhandstück 2, insbesondere mit dem Handgriffabschnitt 4, gekoppelt werden kann oder von diesem entkoppelt werden kann. Das Werkzeug 8 kann dabei beispielsweise als Fräser, Bohrer oder Polierkopf ausgeführt sein. Weiter unten wird die hierfür vorgesehene Schaftkupplungsvorrichtung im Einzelnen beschrieben. Zunächst soll jedoch noch näher auf den Aufbau des Handinstruments 1 eingegangen werden:

Wenn der Schaft 10 mit dem Handgriffabschnitt 4 gekoppelt ist, wird Drehmoment von der Antriebseinheit 6 über einen in dem Handinstrument 1, insbesondere dem Handgriffabschnitt 4 und dem Schaft 10, angeordneten Drehmoment-Übertragungszug hin zu dem Werkzeug 8 übertragen, um dieses in Drehung zu versetzen. Eine Besonderheit des Handinstruments 1 ist darin zu sehen, dass die Achse des Werkzeugs 8 relativ zur Achse des Schafts 10 abgewinkelt werden kann (in Fig. 1 durch den Pfeil C angedeutet). D.h. das Werkzeug 8 und der Schaft 10 können, wie nachstehend näher beschrieben, so zueinander abgewinkelt werden, dass eine Längsachse S1 des Werkzeugs 8 und eine Schaftlängsachse S2 des Schafts 10 miteinander einen Winkel ungleich 180°, insbesondere kleiner als 180°, ausbilden (vgl. Fig. 1).

Wie in Fig. 1 gezeigt, ist hierfür bei dem offenbarungsgemäßen Handinstrument 1 ein hülsenförmiges Bedienelement 12 vorgesehen. Das Bedienelement 12 ist dabei an einem distalen Endabschnitt des Handgriffabschnitts 4 angeordnet und um eine Handgrifflängsachse in einer ersten Drehrichtung A und in einer der ersten Drehrichtung A entgegengesetzten, zweiten Drehrichtung B drehbar. Mit anderen Worten ausgedrückt, weist das Motorhandstück 2 den Handgriffabschnitt 4 und das distal angeordnete Bedienelement 12 auf, welche koaxial zueinander angeordnet sind, wobei das Bedienelement 12 relativ zu dem Handgriffabschnitt 4 gedreht werden kann.

Eine Drehung des Bedienelements 12 aus einer neutralen Nullstellung, bei welcher das Werkzeug 8 nicht relativ zu dem Schaft 10 abgewinkelt ist, in der ersten Drehrichtung A bewirkt dabei, wie in Fig. 1 gezeigt, ein Abwinkeln der Achse des Werkzeugs 8 relativ zu dem Schaft 10. Die erste Drehrichtung A ist bei dem Handinstrument 1 gemäß der vorliegenden Offenbarung als eine Drehung des Bedienelements 12 relativ zu dem Handgriffabschnitt 4 nach links definiert. Anders ausgedrückt entspricht die erste Drehrichtung A in einer distalen Draufsicht auf das Werkzeug 8 einer Drehung des Bedienelements 12 im Uhrzeigersinn.

Wie in Fig. 2 gezeigt, kann das Bedienelement 12 bei dem offenbarungsgemäßen Handinstrument 1 auch in der zweiten Drehrichtung **B,** welche der ersten Drehrichtung A entgegengesetzt ist, relativ zu dem Handgriffabschnitt 4 gedreht werden. D.h. die zweite Drehrichtung B ist in der Draufsicht auf das Werkzeug 8 als eine Drehung des Bedienelements 12 entgegen dem Uhrzeigersinn bzw. als eine Drehung nach rechts definiert. Wie durch den Pfeil D in Fig. 2 angedeutet, bewirkt eine Drehung des Bedienelements 12 in der zweiten Drehrichtung B ein Lösen der Kopplung des Werkzeugs 8 mit dem Schaft 10. Somit kann, wie nachstehend näher erläutert, durch eine Drehung des Bedienelements 12 in der zweiten Drehrichtung C ein einfacher und schneller Werkzeugwechsel gewährleistet werden.

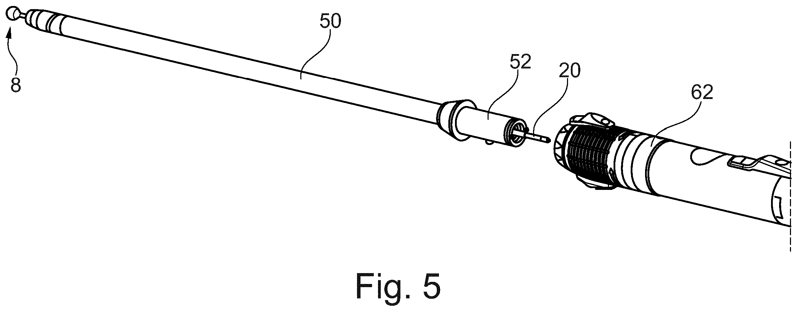

Zusätzlich ist das Handinstrument mit einer nachfolgend näher zu beschreibenden Einrichtung zur lösbaren Ankupplung des Schafts 10, genauer gesagt eines Schaftrohrs 50 an das Motorhandstück 2 ausgestattet. Diese Einrichtung wird nachfolgend als Schaftkupplung bezeichnet. Der abgekuppelte Zustand des Schafts 10 mit innenliegender Antriebswelle 20 ist in Figur 5 gezeigt.

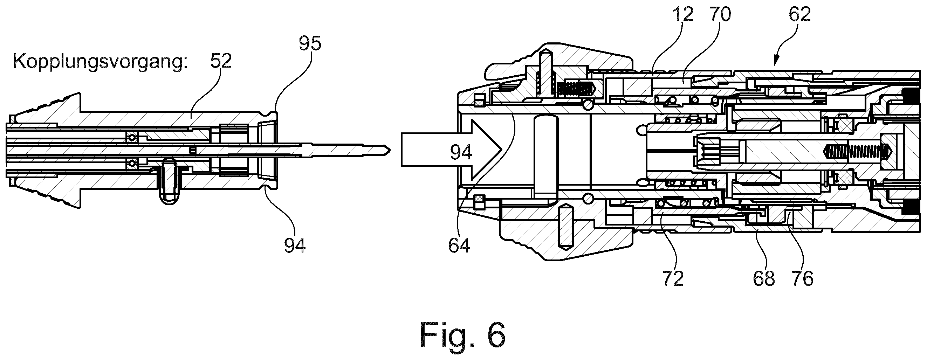

Auf die Einzelheiten der Mechanik zur Abwinkelung und zum Auswerfen der Werkzeugachse muss hier nicht besonders eingegangen werden. Ausschlaggebend für die vorliegende Offenbarung ist, dass die hierfür erforderliche verhältnismäßig komplexe Mechanik - wie in Figur 6 erkennbar - im Inneren eines Schaftrohrs 50 mit einem Kupplungshohlzylinder 52 bzw. innerhalb eines distalen Gehäuseabschnitts 62 des Handgriffabschnitts 4 angeordnet ist, so dass für eine Mechanik zur Ausbildung der Schaftkupplung und ihrer Bedienungselemente ein verhältnismäßig kleiner Bauraum vorgegeben ist.

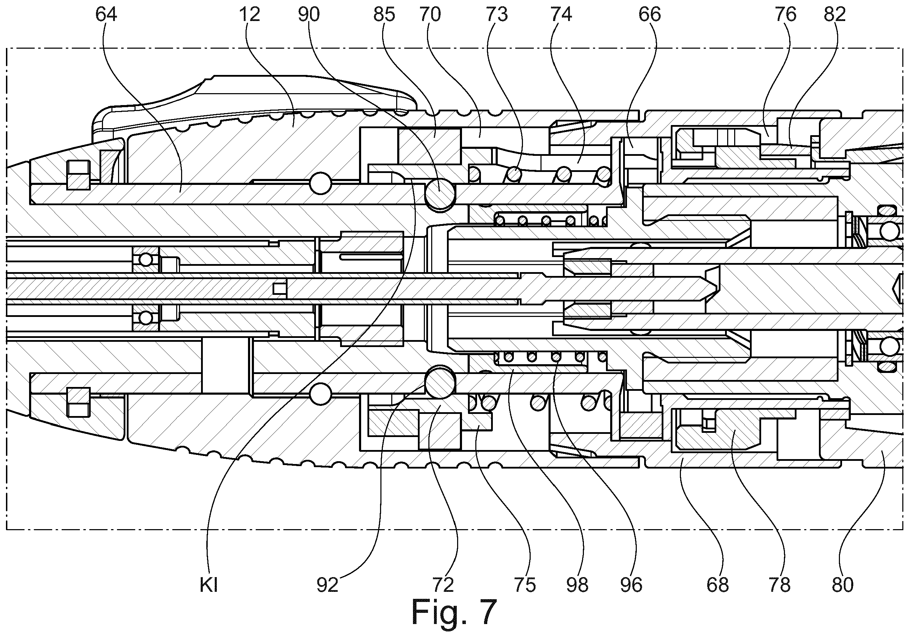

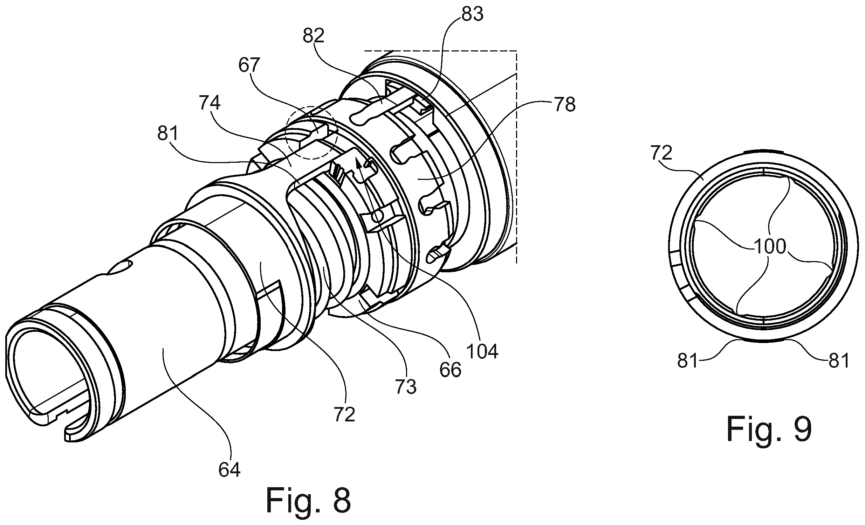

Der Gehäuseabschnitt 62 hat einen Gehäuseträgerkörper, der - wie am besten aus der Figur 8 ersichtlich - einen das Bedienelement 12 drehbar lagernden distalen beispielhaft als Aufnahmezylinder ausgebildeten Aufnahme 64 mit zwei radial nach außen offenen Durchbrüchen 67 aufweisenden Ringflansch 66 ausbildet, über den sich eine das Bedienelement 12 zusätzlich abstützende Zwischenhülse 68 des Gehäuseabschnitts 62 erstreckt. Somit verbleibt auf der distalen Seite des Ringflanschs 66 ein erster Ringraum 70 zur Aufnahme eines einen ersten Bestandteil einer Schaftkupplung ausbildenden Schließhülsenkörpers 72, der über den Umfang verteilt zwei achsparallel ausgerichtete Mitnehmerarme bzw. -zungen 74 mit Gleitflächen 81 hat und der von einer Druckfeder 73 in eine in Figur 6 und 7 gezeigte Anschlagstellung drückbar ist. Der Schließhülsenkörper 72 hat folglich eine sehr geringe radiale Erstreckung. Gleichzeitig dient er als Träger eines mit dem Bezugszeichen 85 bezeichneten Rastrings (siehe Figur 7) für einen nicht näher zu beschreibenden Mechanismus zur Indexierung der Drehbewegung des Bedienelements 12.

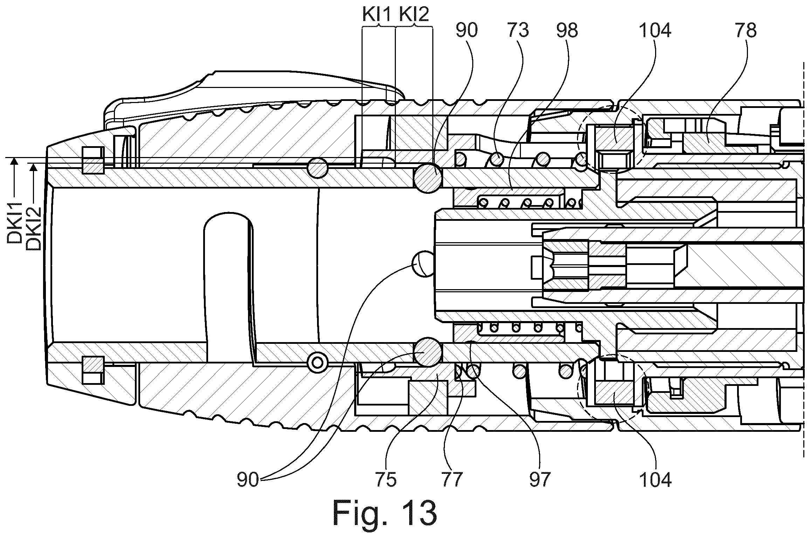

Die Mitnehmerzungen 74 erstrecken sich genauer gesagt von einem Schulterabschnitt 75 des hohlzylindrisch ausgebildeten Schließhülsenkörpers 72 weg, wobei der Schulterabschnitt 75 radial innerkalb der Mitnehmerzunge 74 eine Anlagefläche 77 (siehe Figur 13) für die Druckfeder 73 ausbildet

Einen weiteren Bestandteil der Schaftkupplung bildet zumindest ein Verriegelungskörper 90, der in einer entsprechenden beispielhaft als Ausnehmung ausgebildeten Führungsöffnung 92 der beispielhaft als Aufnahmezylinder ausgebildeten Aufnahme 64 derart formschlüssig aufgenommen ist, dass er durch die Innenkontur KI des Schließhülsenkörpers 72 über die Innenoberfläche der als Aufnahmezylinder ausgebildeten Aufnahme 64 hinaus in eine Kupplungsausnehmung 94 (siehe Figur 6) des Kupplungshohlzylinders 52 drückbar ist.

Im gezeigten Ausführungsbeispiel sind vier beispielhaft als Kugeln ausgebildete und über den Umfang vorzugsweise gleichmäßig verteilte Verriegelungskörper 90 vorgesehen, die vorzugsweise verliersicher - wie in Figur 13 angedeutet - in beispielhaft als radiale Durchbrüche oder Bohrungen ausgebildeten Führungsöffnungen 92 aufgenommen sind und mit einer beispielhaft als Ringnut ausgebildeten Kupplungsausnehmung 94 des Kupplungshohlzylinders 52 zusammenwirken.

Die rotationssymmetrische Innenkontur KI des Schließhülsenkörpers 72 hat - wie am besten aus Figur 7 und 13 ersichtlich - zwei Funktionsabschnitte KI1 und KI2 mit unterschiedlichem Innendurchmesser DKI1 und DKI2 (siehe Figur 13). Der Funktionsabschnitt KI2 mit kleinerem Innendurchmesser DKI2 kann die Kugeln 90 radial nach Innen in die als Ringnut ausgebildete Kupplungsausnehmung 94 des Kupplungshohlzylinders 52 drücken, während der Innendurchmesser DKI1 des Funktionsabschnitts KI1 groß genug ist, um den Kugeln 90 eine behinderungsfreie radiale Bewegung nach außen zu erlauben. Die Druckfeder 73 spannt den Schließhülsenkörper 72 in die Anschlagstellung vor, in der der Funktionsabschnitt KI2 den Kugeln 90 gegenüberliegt.

Im Folgenden wird beschrieben, wie die Betätigung der Schaftkupplung und deren Montage erfolgt:
Die Mitnehmerzungen 74 erstrecken sich durch jeweils einen zugeordneten Durchbruch 67 des Ringflanschs 66 in einen zweiten Ringraum 76 auf der proximalen Seite des Ringflanschs 66. Dieser zweite Ringraum 76 liegt somit radial innerhalb der Zwischenhülse 68 des Gehäuseabschnitts 62 und er nimmt eine von einem beispielhaft als Schieber ausgebildeten Betätigungselement 80 über einen Zugstab 83 (siehe Figur 8) und eine Mitnehmerlasche 82 betätigbare Zughülse 78 auf. Die Zughülse 78 kann mit dem proximalen Ende der Mitnehmerzungen 74 verhakt werden, so dass durch eine Schiebebewegung des Schiebers 80 zur proximalen Seite - wie in Figur 4 mit dem Pfeil ENT angedeutet - der Schließhülsenkörper 72 gegen die Kraft der Druckfeder 73 zur proximalen Seite des Motorhandstücks 2 gezogen wird, bis der Funktionsabschnitt KI1 gegenüber den Kugeln 90 zu liegen kommt und letztere damit radial nach außen frei beweglich werden. Der in Figur 7 gezeigte gekuppelte Zustand des Schafts 10 kann damit aufgehoben werden.

Zur Unterstützung des Abkupplungsvorgangs kann zusätzlich ein von einer Ausstoßfeder 96 beaufschlagter Fang- und Druckkörper 98 vorgesehen sein, der im angekuppelten Zustand des Schafts 10 (siehe Figur 7) an einer Stirnseite des Kupplungshohlzylinders 52 anliegt und einem endseitigen Ringbund 95 des Kupplungshohlzylinders 52 bei der Bewegung an den Kugeln 90 vorbei folgt. Die Kugeln 90 können somit auch dann nicht aus den beispielhaft als Bohrungen ausgeführten Führungsöffnungen 92 fallen, wenn die Bohrungen durchgehend kreiszylindrisch ausgebildet sind. Eine flache Ringnut 97 im Fang- und Druckkörper 98 sichert letzteren im Motorhandstück gegen Herausfallen.

Aus den Figuren und der vorstehenden Beschreibung geht hervor, dass die Ringräume 70, 76 bedingt durch einen handhabungsbedingt vorgegebenen Außendurchmesser des Handstücks und durch die komplexe Mechanik im Inneren des Motorhandstücks 2 bzw. des Handgriffabschnitts 4 in radialer Richtung sehr begrenzt sind. Bei einem Motorhandstück 2 für ein chirurgisches Instrument wie in den Figuren dargestellt, liegt die radiale Erstreckung des Ringraums 70 bzw. 76 lediglich im mm-Bereich. Die vorstehend beschriebenen Komponenten der Schaftkupplung sind demnach so ausgebildet, dass sie eine radial äußerst kompakte Bauweise erlauben, die durch die nachfolgend zu beschreibende Montage der Komponenten unterstützt wird:
Anhand der Figuren 8 bis 13 wird gezeigt, wie die einzelnen Komponenten im Motorhandstück 2 bzw. im Handgriffabschnitt 4 montiert werden. Figur 8 zeigt den Zustand, in dem der Gehäuseabschnitt 62 mit Ausnahme der Zwischenhülse 68 und des Bedienelements 12 fertig montiert ist, so dass der Schieber 80 samt Mitnehmerlasche 82, Zugstab 83 und Zughülse 78 bereits im Gehäuse, also im gezeigten Ausführungsbeispiel im Gehäuseabschnitt 62 aufgenommen sind.

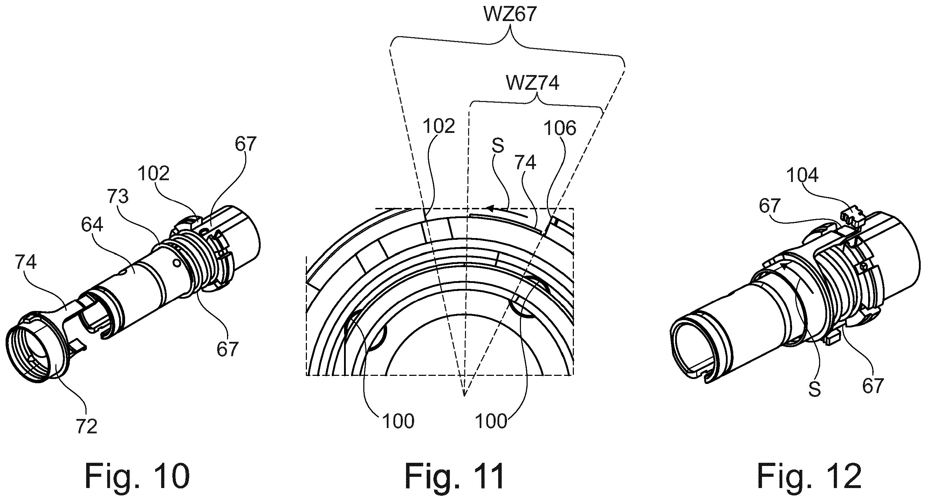

In diesem Montagezustand kann zunächst die Druckfeder 73 (siehe Figur 10) und daraufhin der Schließhülsenkörper 72 auf deie als Aufnahmezylinder ausgebildete Aufnahme 64 des Gehäuseträgerkörpers aufgefädelt werden. Damit jetzt allerdings eine Montage der Kugeln 90 ermöglicht ist, ohne die Baulänge der Druckfeder 73 allzu groß zu machen bzw. ohne die Druckfeder 73 bei der Montage belasten zu müssen, sind folgende Vorkehrungen getroffen:
Im Schließhülsenkörper 72 sind - wie aus der Stirnansicht gemäß Figur 9 erkennbar - in einem Winkelabstand und Muster, das dem Muster der die Kugeln 90 aufnehmenden und beispielhaft als Bohrungen ausgebildeten Führungsöffnungen 92 entspricht, in der Innenoberfläche durchgehende Nuten 100 ausreichender Tiefe ausgebildet, so dass der Schließhülsenkörper 72 zunächst in einer bestimmten Drehlage, die in Figur 10 und 11 gezeigt ist, über die bereits eingelegten, und über den Außenumfang der beispielhaft als Aufnahmezylinder ausgebildeten Aufnahme 64 vorragenden Kugeln 90 geschoben werden kann. Die Tiefe der Nuten 100 wird vorzugsweise so gewählt, dass - wie in Figur 11 dargestellt - der Nutgrund mit dem Innendurchmesser DKI2 des Funktionsabschnitts KI2 fluchtet.

Die Mitnehmerzungen 74 greifen dabei - wie in Figur 11 und 12 dargestellt - durch zugehörige Durchbrüche 67 im Ringflansch 66 des Gehäuseträgerkörpers. Die Seitenwände der Durchbrüche 67 sind mit 102 und 106 bezeichnet, und vorzugsweise wird der Schließhülsenkörper 72 in der anfänglichen Drehlage durch eine erste Seitenwand 106 des Durchbruchs 67 positioniert und geführt. Die Durchbrüche 67 erstrecken sich - wie in Figur 11 dargestellt - über einen Zentriwinkel WZ67, der größer ist als der von den Mitnehmerzungen 74 überstrichene Zentriwinkel WZ74. Sobald der Schließhülsenkörper 72 - zuletzt gegen eine geringe Druckkraft der Druckfeder 73 - so weit aufgefädelt ist, dass die Nuten 100 die Kugeln 90 passiert haben, kann er - wie mit dem Pfeil S in Figur 11 und 12 markiert - so weit verdreht werden, bis die Mitnehmerzungen 74 an einer zweiten Seitenwand 102 des Durchbruchs 67 anschlagen. Dadurch verlagern sich auch die Nuten 100 in Umfangsrichtung und liegen winkelmäßig versetzt zu den Kugeln 90, die dadurch den Schließhülsenkörper 72 axial im Gehäuseabschnitt 62 fixieren. Diese Drehbewegung des Schließhülsenkörpers 72 kann vorteilhafterweise auch dazu genutzt werden, die proximalen Endabschnitte der Mitnehmerzungen 74 mit der Zughülse 78 zu verhaken.

Mittels passgenau gefertigter beispielhaft als Einlegeteile ausgebildeter Passteile 104, die verzahnungsähnlich wie ein Puzzleteil in die andere, zweite Seitenwand 106 des Durchbruchs 67 eingefügt werden, werden die Mitnehmerzungen 74 verdrehsicher am Gehäuseträgerkörper des Gehäuseabschnitts 62 fixiert. Die Verzahnung zwischen Passteil 104 und Seitenwand 106 hat den besonderen Vorteil, dass die Passteile 104 keine zusätzlichen Befestigungsmittel benötigen und allein durch den Passungseingriff mit der Wand 106 im Durchbruch 67 für eine saubere axiale Führung der Mitnehmerzungen 74 sorgen und dabei mit den Gleitflächen 75 am Passteil 104 und an der zweiten Seitenwand 102 des Durchbruchs 67 anliegen.

Eine passgenaue Verzahnungsflächenpaarung zwischen den Passteilen 104 und der Seitenwand 106 des Durchbruchs 67 lässt sich beispielsweise dadurch mit geringem Aufwand herstellen, dass zumindest im Bereich des Ringflanschs 66 zur Ausbildung des Verzahnungsprofils der Seitenwand 106 ein Drahterosionsverfahren zur Anwendung kommt.

In dieser Montagesituation kann - wie aus den Figuren 6, 7 und 13 ersichtlich - die Zwischenhülse 68 auf den Gehäuseabschnitt 62 geschraubt werden, wodurch die Passteile 104 gesichert werden, ohne dass es dazu irgendwelcher weiterer Hilfsmittel, wie z.B. Klebstoff oder dgl., bedarf. Hierdurch wird die Montage und die Demontage erheblich erleichtert.

Aus der vorstehenden Beschreibung wird klar, dass die Druckfeder 73 bei der Montage der Schaftkupplung keiner zusätzlichen Belastung unterworfen wird, und es auch möglich bleibt, die axiale Baulänge der Druckfeder 73 kompakt bzw. klein zu halten.

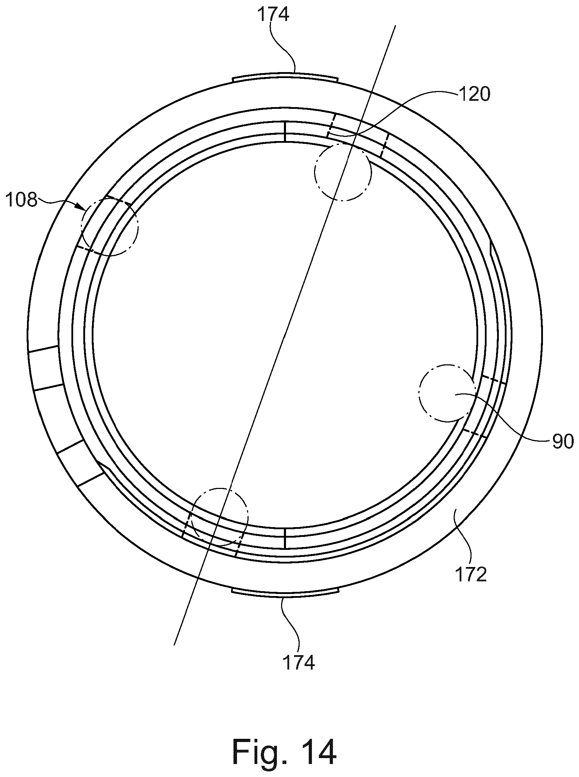

Alternativ zu der vorstehend beschriebenen Montage der Schaftkupplung ist es unter Beibehaltung der beschriebenen Vorteile auch möglich, den zumindest einen Verriegelungskörper 90, also für das gezeigte Ausführungsbeispiel die Verriegelungskörper 90 in der Ausgestaltung als Kugeln 90, von außen über zugeordnete, radial ausgerichtete Befüllöffnungen 108 durch den Schließhülsenkörper 72 einzulegen. Diese Variante wird unter Bezugnahme auf die Figur 14 näher beschrieben, die eine vergrößerte Stirnansicht des Schließhülsenkörpers zeigt.

Der Schließhülsenkörper 172 dieser Modifikation hat dann anstelle der oben beschriebenen Nuten 100, eine im selben Winkelabstandsmuster angeordnete, entsprechende Anzahl von Radialbohrungen 120, durch die die Verriegelungskörper, beispielsweise in der Ausgestaltung von Kugeln 90, die in Figur 14 mit strichpunktierter Linie angedeutet sind, eingefüllt werden können. Das Einfüllen von außen ist möglich, wenn der Schließhülsenkörper 172 in der Ausrichtung entsprechend Figur 10 so weit auf die beispielhaft als Aufnahmezylinder ausgebildete Aufnahme 64 geschoben ist, dass die Radialbohrungen 120 mit den beispielhaft als Bohrungen ausgebildeten Führungsöffnungen 92 im Aufnahmezylinder fluchten. Der Schließhülsenkörper 172 befindet sich dabei in einer Axialposition wie in Figur 7 oder 13 dargestellt und in einer Drehlage entsprechend Figur 11. In dieser Montageposition können die Kugeln 90 in die beispielhaft als Ausnehmungen ausgebildeten Führungsöffnungen 92 der beispielhaft als Aufnahmezylinder ausgebildeten Aufnahme 64 eingelegt werden, wobei entweder durch eine radiale Verjüngung der beispielhaft als Ausnehmungen ausgebildeten Führungsöffnungen 92 oder durch Fang- und Druckkörper 98 dafür gesorgt wird, dass die Kugeln 90 nicht nach innen fallen.

In dieser Axialposition wird der Schließhülsenkörper 172 wie in Verbindung mit den Figuren 10 bis 12 beschrieben so weit verdreht, dass die Mitnehmerzungen 174 an der ersten Wand des Durchbruchs des Ringflanschs 66 anschlagen. Die Radialbohrungen 120 gelangen hierdurch außer Fluchtung mit den Kugeln 90 und sie liegen damit nicht mehr auf der beim Lösen der Schaftkupplung auftretenden Bewegungsbahn von Schließhülse 172 und Kugeln 90.

Selbstverständlich sind Abweichungen von den beschriebenen Ausführungsformen möglich, ohne den Grundgedanken der Offenbarung zu verlassen. Das Anwendungsgebiet der vorstehend beschriebenen Schaftkupplung ist auch nicht auf ein medizinisches Instrument beschränkt. Der Aufbau und die Montagetechnik der Schaftkupplung ist überall dort sinnvoll und anwendbar, wo es darum geht, die Komponenten der Schaftkupplung auf sehr engem radialen Bauraum unterzubringen mit einfachen Handgriffen zu montieren und bei der Montage mechanisch möglichst wenig zu belasten.

Variationsmöglichkeiten bestehen beispielsweise hinsichtlich der Anzahl und der Form der Verriegelungskörper und hinsichtlich der Mitnehmerzungen.

Der Gegenstand der Offenbarung schafft somit eine Schaftkupplung für die lösbare Ankupplung eines kreiszylindrischen Schafts, beispielsweise eines medizinischen Instruments, an eine kreiszylindrische Aufnahme eines Gehäuses, wie z.B. eines Motorhandstücks eines medizinischen Instruments, das ein Betätigungselement zur Entriegelung der Schaftkupplung trägt. Die Schaftkupplung hat zumindest einen Verriegelungskörper, der in der kreiszylindrischen Aufnahme in einer Führungsöffnung radial beweglich aufgenommen und mittels eines axial beweglichen Schließkörpers in dessen erster Funktionsstellung in einer Sperrstellung fixierbar ist, in der er in eine Kupplungsausnehmung des Schafts formschlüssig eingreift. Die Sperrstellung des Verriegelungskörpers ist aufhebbar ist, indem der Schließkörper gegen die Kraft einer Druckfeder axial in eine die Kupplung entriegelnde zweite Funktionsstellung verschiebbar ist, in der der Verriegelungsköper radial nach außen außer Eingriff mit dem Schaft bewegbar ist. Um die Komponenten der Schaftkupplung auf engstem radialen Bauraum aufzunehmen und die Druckfeder bei der Montage nicht zu beanspruchen, ist der Schließkörper als Schließhülse ausgebildet, die zusammen mit der Druckfeder auf der Außenseite der kreiszylindrischen Aufnahme geführt sitzt und auf der vom Schaft weg gerichteten Seite mit zumindest einer mit dem Betätigungselement koppelbaren Mitnehmerzunge ausgestattet ist. Die Mitnehmerzunge ermöglicht es der Schließhülse, in einer ersten Drehstellung auf die Außenseite der kreiszylindrischen Aufnahme aufgefädelt und in einer zweiten Drehstellung drehfest fixiert zu werden, wobei die Schließhülse eine der Anzahl der Verriegelungskörper entsprechende Anzahl von Montage-Ausnehmungen aufweist, die nur in der ersten Drehstellung der Schließhülse in Umfangsrichtung mit den Führungsöffnungen bzw. mit den Verriegelungskörpern fluchten.

**Bezugszeichenliste**

| | | | |
|---|---|---|---|
| 1 | Handinstrument | | |
| 2 | Motorhandstück | | |
| 4 | Handgriffabschnitt | | |
| 6 | Antriebseinheit | | |
| 7 | Kabel | | |
| 8 | Werkzeug | | |
| 10 | Schaft | S1, S2 | Längsachsen von 8 und 10 |
| 20 | Antriebswelle | | |
| 12 | Bedienelement | | |
| 50 | Schaftrohr | | |
| 52 | Kupplungshohlzylinder | | |
| 62 | distaler Gehäuseabschnitt eines Gehäuses | | |
| 64 | Aufnahme | | |
| 66 | Ringflansch | | |
| 67 | Durchbrüche | WZ67 | Zentriwinkel |
| 68 | Zwischenhülse | | |
| 70 | erster Ringraum | | |
| 72 | Schließkörper | | |
| | Schließhülsenkörper | KI1, KI2 | Funktionsabschnitte |
| | | KI | Innenkontur |
| 73 | Druckfeder | | |
| 74 | Mitnehmerzungen | WZ74 | Zentriwinkel |
| 75 | Schulterabschnitt | | |
| 76 | zweiter Ringraum | | |
| 77 | Anlagefläche | | |
| 78 | Zughülse | | |
| 80 | Betätigungselement in der beispielhaften Ausgestaltung als Schieber | | |
| 81 | Gleitflächen | | |
| 82 | Mitnehmerlasche | | |
| 83 | Zugstab | | |
| 85 | Rastring | | |
| 90 | Verriegelungskörper in der beispielhaften Ausgestaltung als Kugeln | | |
| 92 | Führungsöffnungen in der beispielhaften Ausgestaltung als Ausnehmungen oder Bohrungen | | |
| 94 | Kupplungsausnehmung in der beispielhaften Ausgestaltung als Ringnut | | |
| 95 | Ringbund | | |
| 96 | Ausstoßfeder | | |
| 97 | Ringnut | | |
| 98 | Fang- und Druckkörper | | |
| 100 | Nuten | | |
| 102 | erste Seitenwand | | |
| 104 | Passteile in der beispielhaften Ausgestaltung als Einlegeteile | | |
| 106 | zweite Seitenwand | | |
| 108 | Montageausnehmungen in der beispielhaften Ausgestaltung als Befüllöffnungen | | |
| 120 | Montageausnehmungen in der beispielhaften Ausgestaltung als Radialbohrungen | | |
| 172 | Schließhülsenkörper modifiziert | | |
| 174 | Mitnehmerzungen | | |

## Patentansprüche

1. Schaftkupplung für die lösbare Ankupplung eines kreiszylindrischen Schafts (10, 52), beispielsweise eines medizinischen Instruments, an eine kreiszylindrische Aufnahme (64) eines Gehäuseabschnitts (62), wie z.B. eines Motorhandstücks (2) eines medizinischen Instruments, das ein Betätigungselement (80) zur Entriegelung der Schaftkupplung trägt, mit zumindest einem Verriegelungskörper (90), der in der kreiszylindrischen Aufnahme (64) in einer Führungsöffnung (92) radial beweglich aufgenommen und mittels eines axial beweglichen Schließkörpers (72) in dessen erster Funktionsstellung in einer Sperrstellung fixierbar ist, in der er in eine Kupplungsausnehmung (94) des Schafts (52) formschlüssig eingreift, wobei die Sperrstellung des Verriegelungskörpers (90) aufhebbar ist, indem der Schließkörper (72) gegen die Kraft einer Druckfeder (73) axial in eine die Schaftkupplung entriegelnde zweite Funktionsstellung verschiebbar ist, in der der Verriegelungsköper (90) radial nach außen außer Eingriff mit dem Schaft (52) bewegbar ist, **dadurch gekennzeichnet, dass** der Schließkörper (72) als Schließhülse ausgebildet ist, die zusammen mit der Druckfeder (73) auf der Außenseite der kreiszylindrischen Aufnahme (64) geführt sitzt und auf der vom Schaft (52) weg gerichteten Seite mit zumindest einer mit dem Betätigungselement (80) koppelbaren Mitnehmerzunge (74) ausgestattet ist, die es dem als Schließhülse ausgebildeten Schließkörper (72) ermöglicht, in einer ersten Drehstellung auf die Außenseite der kreiszylindrischen Aufnahme (64) aufgefädelt und in einer zweiten Drehstellung drehfest fixiert zu werden, wobei der als Schließhülse ausgebildete Schließkörper (72) eine der Anzahl der Verriegelungskörper (90) entsprechende Anzahl von Montage-Ausnehmungen (100; 108; 120) aufweist, die nur in der ersten Drehstellung des als Schließhülse ausgebildeten Schließkörpers (72) in Umfangsrichtung mit den Führungsöffnungen (92) fluchten.

2. Schaftkupplung nach Anspruch 1, **dadurch gekennzeichnet, dass** der als Schließhülse ausgebildete Schließkörper (72) in der zweiten Drehstellung drehfest im Gehäuseabschnitt (62) fixierbar ist.

3. Schaftkupplung nach Anspruch 2, **dadurch gekennzeichnet, dass** sich die zumindest eine Mitnehmerzunge (74) durch einen zugehörigen Durchbruch (67) in einem Ringflansch (66) der kreiszylindrischen Aufnahme (64) erstreckt und in der zweiten Drehstellung mittels eines radial von außen einsetzbaren Passteils (104) in einer Anlagestellung an einer Seitenwand (102) des Durchbruchs (67) fixierbar ist.

4. Schaftkupplung nach einem der Ansprüche 1 bis 3, **dadurch gekennzeichnet, dass** der als Schließhülse ausgebildete Schließkörper (72) innenseitig zwei axial hintereinanderliegende Funktionsabschnitte (KI1, KI2) mit unterschiedlichen Innendurchmessern (DKI1, DKI2) ausbildet.

5. Schaftkupplung nach Anspruch 4, **dadurch gekennzeichnet, dass** sich die zumindest eine Mitnehmerzunge (74) von einem Schulterabschnitt (75) eines die Funktionsabschnitte (KI1, KI2) ausbildenden Teils des als Schließhülse ausgebildeten Schließkörpers (72) weg erstreckt, und der Schulterabschnitt (75) radial innerhalb der Mitnehmerzunge (74) eine Anlagefläche (77) für die Druckfeder (73) ausbildet.

6. Schaftkupplung nach einem der Ansprüche 1 bis 5, **dadurch gekennzeichnet, dass** mehrere über den Umfang vorzugsweise gleichmäßig verteilte Verriegelungskörper (90) und/oder Mitnehmerzungen (74) vorgesehen sind.

7. Schaftkupplung nach einem der Ansprüche 1 bis 6, **dadurch gekennzeichnet, dass** der zumindest eine Verriegelungskörper (90) als Kugel ausgebildet ist.

8. Schaftkupplung nach einem der Ansprüche 5 bis 7, **dadurch gekennzeichnet, dass** die zumindest eine Montageausnehmung (100) in dem als Schließhülse ausgebildeten Schließkörper (72) von einer im Schulterabschnitt (75) innenseitig ausgebildeten und axial verlaufenden Nut (100) gebildet ist, die ausreichend tief ist, um den Schulterabschnitt (75) des Schließkörpers (72) axial über den zugeordneten, bereits in der zylindrischen Aufnahme (64) montierten Verriegelungskörper (90) zu schieben.

9. Schaftkupplung nach einem der Ansprüche 1 bis 7, **dadurch gekennzeichnet, dass** die zumindest eine Montageausnehmung (108; 120) von einer jeweils einem Verriegelungskörper (90) zugeordneten Befüllöffnung (108) bzw. Radialbohrung (120) gebildet ist, durch die in der ersten Drehstellung des als Schließhülse ausgebildeten Schließkörpers (72) jeweils ein Verriegelungskörper (90) radial in die Führungsöffnung (92) der zylindrischen Aufnahme (64) einsetzbar ist.

10. Montageverfahren für Komponenten einer Schaftkupplung nach einem der Ansprüche 1 bis 9, bei der ein kreiszylindrischer Schaft (10, 12, 52), beispielsweise eines medizinischen Instruments, an eine kreiszylindrische Aufnahme (64) eines Gehäuseabschnitts (62), wie z.B. eines Motorhandstücks (2) eines medizinischen Instruments, lösbar ankuppelbar ist, wobei der Gehäuseabschnitt (62) ein Betätigungselement (80) zur Entriegelung der Schaftkupplung trägt, und die kreiszylindrische Aufnahme (64) in einer Führungsöffnung (92) radial beweglich zumindest einen Verriegelungskörper (90) aufnimmt, der mittels eines axial beweglichen Schließkörpers (72) in dessen erster Funktionsstellung in einer Sperrstellung fixierbar ist, in der er in eine Kupplungsausnehmung (94) des Schafts (52) formschlüssig eingreift, wobei die Sperrstellung des Verriegelungskörpers (90) aufhebbar ist, indem der Schließkörper (72) gegen die Kraft einer Druckfeder (73) axial in eine die Schaftkupplung entriegelnde zweite Funktionsstellung verschiebbar ist, in der der Verriegelungsköper (90) radial nach außen außer Eingriff mit dem Schaft bewegbar ist, und wobei der Schließkörper (72) als Schließhülse ausgebildet ist, die zusammen mit der Druckfeder (73) auf der Außenseite der kreiszylindrischen Aufnahme (64) geführt sitzt und auf der vom Schaft (52) weg gerichteten Seite mit zumindest einer mit dem Betätigungselement (80) koppelbaren Mitnehmerzunge (74) ausgestattet ist, **dadurch gekennzeichnet, dass** der als Schließhülse ausgebildete Schließkörper (72) zusammen mit der Druckfeder (73) in einer ersten Drehstellung, in der eine der Anzahl der Verriegelungskörper (90) entsprechende Anzahl von Montage-Ausnehmungen (100; 108; 120) in Umfangsrichtung mit den Führungsöffnungen (92) fluchten, auf die Außenseite der kreiszylindrischen Aufnahme (64) aufgefädelt und in einer zweiten Drehstellung drehfest fixiert wird.

11. Montageverfahren nach Anspruch 10, bei dem der zumindest eine Verriegelungskörper (90) vor dem Auffädeln des als Schließhülse ausgebildeten Schließkörpers (72) bereits in die zugehörige Führungsöffnung (92) eingelegt wird, und der Schließkörper (72), der radial innenseitig mit einer der Anzahl der Verriegelungskörper (90) entsprechenden Anzahl von axial verlaufenden Nuten (100) ausgestattet ist, in der ersten Drehstellung axial über die zugeordneten Verriegelungskörper (90) geschoben und anschließend in die zweite Drehstellung verdreht und über die zumindest eine Mitnehmerzunge (74) in dieser Drehstellung durch ein von außen eingesetztes Passteil (104) fixiert wird.

12. Montageverfahren nach Anspruch 10, bei dem der zumindest eine Verriegelungskörper (90) nach dem Auffädeln des als Schließhülse ausgebildeten Schließkörpers (72) in deren erster Drehstellung in eine zugeordnete radiale Befüllöffnung (108) bzw. Radialbohrung (120) eingelegt wird, und der Schließkörper (72) anschließend in die zweite Drehstellung verdreht und über die zumindest eine Mitnehmerzunge (74) in dieser Drehstellung durch ein von außen eingesetztes Passteil (104) fixiert wird.

13. Montageverfahren nach Anspruch 11 oder 12, **dadurch gekennzeichnet, dass** das Passteil (104) durch eine auf den Gehäuseabschnitt (62) geschraubte Zwischenhülse (68) radial gesichert wird.

## Claims

1. A shaft coupling for detachable connection of a circular cylindrical shaft (10, 52), for example, of a medical instrument, to a circular cylindrical receptacle (64) of a housing portion (62), such as of a motor handpiece (2) of a medical instrument, which carries an actuating element (80) for unlocking the shaft coupling, with at least one locking body (90), which is received radially movably in the circular cylindrical receptacle (64) in a guide opening (92) and is fixable via an axially movable closure body (72) in its first functional position in a locking position, in which it engages form-fittingly in a coupling recess (94) of the shaft (52), wherein the locking position of the locking body (90) can be released by the closure body (72) being axially displaceable against the force of a compression spring (73) into a second functional position, in which the locking body (90) is movable radially outward out of engagement with the shaft (52), **characterized in that** the closure body (72) is configured as a closure sleeve which, together with the compression spring (73), is seated in a guided manner on the outside of the circular cylindrical receptacle (64) and, on the side facing away from the shaft (52), is equipped with at least one follower tongue (74) which can be coupled to the actuating element (80) and which allows the closure body (72) formed as closure sleeve to be threaded onto the outside of the circular cylindrical receptacle in a first rotational position and to be fixed against rotation in a second rotational position, wherein the closure body (72) formed as closure sleeve has a number of mounting recesses (100; 108,120) corresponding to the number of locking bodies (90), said mounting recesses (100; 108,120) being aligned in a circumferential direction with the guide openings (92) only in the first rotational position of the closure body (72) formed as closure sleeve.

2. The shaft coupling according to claim 1, **characterized in that** the closure body (72) formed as closure sleeve is fixable in the housing portion (62) in a rotationally fixed manner in the second rotational position.

3. The shaft coupling according to claim 2, **characterized in that** the at least one follower tongue (74) extends through an associated aperture (67) in a ring flange (66) of the circular cylindrical receptacle (64) and is fixable in the second rotational position in a contact position on a sidewall (102) of the aperture (67) via a fitting part (104) that is insertable radially from outside.

4. The shaft coupling according to any of claims 1 to 3, **characterized in that**, on an inner side, the closure body (72) formed as closure sleeve forms two axially successive functional portions (KI1, KI2) with different inside diameters (DKI1, DKI2).

5. The shaft coupling according to claim 4, **characterized in that** the at least one follower tongue (74) extends away from a shoulder portion (75) of a part of the closure body (72) formed as closure sleeve forming the functional portions (KI1, KI2), and the shoulder portion (75) forms a contact surface (77) for the compression spring (73) radially inside the follower tongue (74).

6. The shaft coupling according to any of claims 1 to 5, **characterized in that** a plurality of locking bodies (90) and/or follower tongues (74) is provided, evenly distributed over the circumference.

7. The shaft coupling according to any of claims 1 to 6, **characterized in that** the at least one locking body (90) is configured as a ball.

8. The shaft coupling according to any of claims 5 to 7, **characterized in that** the at least one mounting recess (100) in the closure body (72) formed as closure sleeve is formed by an axially extending groove (100) formed in the shoulder portion (75) on the inside, wherein the groove (100) is sufficiently deep to slide the shoulder portion (75) of the closure body (72) axially over the associated locking body (90) already mounted in the cylindrical receptacle (64).

9. The shaft coupling according to any of claims 1 to 7, **characterized in that** the at least one mounting recess (108, 120) is formed by a respective filler opening (108) or radial bore (120) assigned to each locking body (90) and through which the respective locking body (90) is insertable radially into the guide opening (92) of the cylindrical receptacle (64) in the first rotational position of the closure body (72) formed as closure sleeve.

10. A mounting method for components of a shaft coupling according to any of claims 1 to 9, wherein a circular cylindrical shaft (10, 12, 52), for example, of a medical instrument, is detachably connectable to a circular cylindrical receptacle (64) of a housing portion (62) such as of a motor handpiece (2) of a medical instrument, the housing portion (62) carrying an actuating element (80) for unlocking the shaft coupling, and the circular cylindrical receptacle (64) radially movably receives at least one locking body (90) in a guide opening (92) and is fixable via an axially movable closure body (72) in its first functional position in a locking position, in which it engages form-fittingly in a coupling recess (94) of the shaft (52), wherein the locking position of the locking body (90) can be released by the closure body (72) being axially displaceable against the force of a compression spring (73) into a second functional position, which unlocks the shaft coupling and in which the locking body (90) is movable radially outward out of engagement with the shaft, and wherein the closure body (72) is configured as a closure sleeve which, together with the compression spring (73), is seated guided on the outside of the circular cylindrical receptacle (64) and, on the side facing away from the shaft (52), is equipped with at least one follower tongue (74) which can be coupled to the actuating element (80), **characterized in that** the closure body (72) formed as closure sleeve together with the compression spring (73), in a first rotational position, in which a number of mounting recesses (100; 108, 120), corresponding to the number of the locking bodies (90), are aligned in circumferential direction with the guide openings (92), are threaded onto the outer side of the circular cylindrical receptacle (64) and are fixed against rotation in a second rotational position.

11. The mounting method according to claim 10, in which the at least one locking body (90) is already inserted into the associated guide opening (92) prior to threading of the closure body (72) formed as closure sleeve, and the closure body (72), which is provided radially on the inside with a number of axially extending grooves (100) corresponding to the number of the locking bodies (90), is pushed axially over the associated locking bodies (90) in the first rotational position and then twisted into the second rotational position and fixed in this rotational position via the at least one follower tongue (74) by a fitting part (104) inserted from the outside.

12. The mounting method according to claim 10, in which the at least one locking body (90) is inserted into an associated radial filler opening (108) or a radial bore (120) after threading of the closure body (72) formed as closure sleeve in its first rotational position, and the closure body (72) is subsequently twisted into the second rotational position and fixed in this rotational position via the at least one follower tongue (74) by a fitting part (104) inserted from the outside.

13. The mounting method according to claim 11 or 12, **characterized in that** the fitting part (104) is radially secured by an intermediate sleeve (68) screwed onto the housing portion (62).

## Revendications

1. Accouplement d'arbre pour le couplage amovible d'un arbre cylindrique circulaire (10, 52), par exemple d'un instrument médical, au niveau d'un logement cylindrique circulaire (64) d'une section de boîtier (62), comme par ex. d'une pièce à main motorisée (2) d'un instrument médical qui porte un élément d'actionnement (80) destiné au déverrouillage de l'accouplement d'arbre avec au moins un corps de verrouillage (90) qui est logé radialement de manière mobile dans le logement cylindrique circulaire (64) dans une ouverture de guidage (92) et peut être fixé dans une position de blocage au moyen d'un corps de fermeture (72) mobile axialement dans la première position fonctionnelle de celui-ci, position de blocage dans laquelle il s'engrène par complémentarité de formes dans un évidement d'accouplement (94) de l'arbre (52), dans lequel la position de blocage du corps de verrouillage (90) peut être annulée en déplaçant le corps de fermeture (72) axialement dans une seconde position fonctionnelle déverrouillant l'accouplement d'arbre contre la force d'un ressort de compression (73), position fonctionnelle dans laquelle le corps de verrouillage (90) est mobile radialement vers l'extérieur pour se désengager de l'arbre (52), **caractérisé en ce que** le corps de fermeture (72) est réalisé en tant que douille de fermeture qui, conjointement avec le ressort de compression (73), est guidée et logée sur le côté extérieur du logement cylindrique circulaire (64) et est équipé sur le côté opposé à l'arbre (52) d'au moins une languette d'entraînement (74) pouvant être accouplée à l'élément d'actionnement (80), languette qui permet au corps de fermeture (72) réalisé en tant que douille de fermeture d'être enfilé dans une première position de rotation sur le côté extérieure du logement cylindrique circulaire (64) et d'être fixé sans pouvoir tourner dans une seconde position de rotation, dans lequel le corps de fermeture (72) réalisé en tant que douille de fermeture présente un nombre d'évidements de montage (100 ; 108 ; 120) correspondant au nombre de corps de verrouillage (90) qui sont alignés avec les ouvertures de guidage (92) dans la direction périphérique uniquement dans la première position de rotation du corps de fermeture (72) réalisé en tant que douille de fermeture.

2. Accouplement d'arbre selon la revendication 1, **caractérisé en ce que** le corps de fermeture (72) réalisé en tant que douille de fermeture peut être fixé sans pouvoir tourner dans la section de boîtier (62) dans la seconde position de rotation.

3. Accouplement d'arbre selon la revendication 2, **caractérisé en ce que** l'au moins une languette d'entraînement (74) s'étend dans une bride annulaire (66) du logement cylindrique circulaire (64) à travers une percée (67) correspondante et peut être fixée à une paroi latérale (102) de la percée (67) dans la seconde position de rotation au moyen d'une pièce d'ajustage (104) qui peut être insérée radialement depuis l'extérieur.

4. Accouplement d'arbre selon l'une quelconque des revendications 1 à 3, **caractérisé en ce que** le corps de fermeture (72) réalisé en tant que douille de fermeture réalise à l'intérieur deux sections fonctionnelles (KI1, KI2) disposées axialement l'une derrière l'autre avec des diamètres intérieurs différents (DKI1, DKI2).

5. Accouplement d'arbre selon la revendication 4, **caractérisé en ce que** l'au moins une languette d'entraînement (74) s'étend à distance d'une section d'épaulement (75) d'une partie formant les sections fonctionnelles (KI1, KI2) du corps de fermeture (72) réalisé en tant que douille de fermeture, et la section d'épaulement (75) réalise une surface de butée (77) pour le ressort de compression (73) radialement à l'intérieur de la languette d'entraînement (74).

6. Accouplement d'arbre selon l'une quelconque des revendications 1 à 5, **caractérisé en ce que** plusieurs corps de verrouillage (90) et/ou languettes d'entraînement (74) répartis de préférence de manière uniforme sur la circonférence sont prévus.

7. Accouplement d'arbre selon l'une quelconque des revendications 1 à 6, **caractérisé en ce que** l'au moins un corps de verrouillage (90) est réalisé en tant que sphère.

8. Accouplement d'arbre selon l'une quelconque des revendications 5 à 7, **caractérisé en ce que** l'au moins un évidement de montage (100) est formé dans le corps de fermeture (72) réalisé en tant que douille de fermeture par une rainure (100) réalisée dans la section d'épaulement (75) et s'étendant axialement, laquelle rainure est suffisamment profonde pour pousser la section d'épaulement (75) du corps de fermeture (72) axialement par le biais du corps de verrouillage (90) associé déjà monté dans le logement cylindrique (64).

9. Accouplement d'arbre selon l'une quelconque des revendications 1 à 7, **caractérisé en ce que** l'au moins un évidement de montage (108 ; 120) est formé par une ouverture de remplissage (108) ou un alésage radial (120) associé respectivement à un corps de verrouillage (90), ouverture ou alésage à travers lesquels respectivement un corps de verrouillage (90) peut être inséré radialement dans l'ouverture de guidage (92) du logement cylindrique (64) dans la première position de rotation du corps de fermeture (72) réalisé en tant que douille de fermeture.

10. Procédé de montage pour des composants d'un accouplement d'arbre selon l'une quelconque des revendications 1 à 9, dans lequel un arbre cylindrique circulaire (10, 12, 52), par exemple d'un instrument médical, peut être accouplé de manière amovible à un logement cylindrique circulaire (64) d'une section de boîtier (62), comme par ex. d'une pièce à main motorisée (2) d'un instrument médical, dans lequel la section de boîtier (62) porte un élément d'actionnement (80) destiné au déverrouillage de l'accouplement d'arbre, et le logement cylindrique circulaire (64) loge radialement de manière mobile dans une ouverture de guidage (92) au moins un corps de verrouillage (90) qui peut être fixé dans une position de blocage au moyen d'un corps de fermeture mobile axialement (72) dans la première position fonctionnelle de celui-ci, position fonctionnelle dans laquelle il s'engage par complémentarité de formes dans un évidement d'accouplement (94) de l'arbre (52), dans lequel la position de blocage du corps de verrouillage (90) peut être annulée en déplaçant le corps de fermeture (72) axialement dans une seconde position fonctionnelle déverrouillant l'accouplement d'arbre contre la force d'un ressort de compression (73), position fonctionnelle dans laquelle le corps de verrouillage (90) est mobile radialement vers l'extérieur pour se désengager de l'arbre et dans lequel le corps de fermeture (72) est réalisé en tant que douille de fermeture qui, conjointement avec le ressort de compression (73) est guidé et logé sur le côté extérieur du logement cylindrique circulaire (64) et est équipé sur le côté opposé à l'arbre (52) d'au moins une languette d'entraînement (74) pouvant être accouplée à l'élément d'actionnement (80), **caractérisé en ce que** le corps de fermeture (72) réalisé en tant que douille de fermeture est aligné conjointement avec le ressort de compression (73) dans une première position de rotation dans laquelle un nombre d'évidements de montage (100 ; 108 ; 120) correspondant au nombre de corps de verrouillage (90) s'alignent dans la direction circonférentielle avec les ouvertures de guidage (92), est enfilé sur le côté extérieur du logement cylindrique circulaire (64) et est fixé sans pouvoir tourner dans une seconde position de rotation.

11. Procédé de montage selon la revendication 10, dans lequel l'au moins un corps de verrouillage (90) est déjà inséré dans l'ouverture de guidage (92) correspondante avant l'enfilage du corps de fermeture (72) réalisé en tant que douille de fermeture, et le corps de fermeture (72) qui est équipé radialement à l'intérieur d'un nombre de rainures (100) s'étendant axialement correspondant au nombre des corps de verrouillage (90), est poussé dans la première position de rotation axialement par le biais des corps de verrouillage (90) associés et ensuite tourné dans la seconde position de rotation, et une pièce d'ajustage (104) insérée depuis l'extérieur est fixée dans cette position de rotation par le biais de l'au moins une languette d'entraînement (74).

12. Procédé de montage selon la revendication 10, dans lequel l'au moins un corps de verrouillage (90) est inséré après l'enfilage du corps de fermeture (72) réalisé en tant que douille de fermeture dans la première position de rotation de celui-ci dans une ouverture de remplissage radiale (108) ou un alésage radial (120) associés, et le corps de fermeture (72) est ensuite tourné dans la seconde position de rotation et une pièce d'ajustage (104) insérée depuis l'extérieur dans cette position de rotation est fixée par le biais de l'au moins une languette d'entraînement (74).

13. Procédé de montage selon la revendication 11 ou 12, **caractérisé en ce que** la pièce d'ajustage (104) est bloquée radialement par le biais d'une douille intermédiaire (68) vissée sur la section de boîtier (62).
